# EUROPÄISCHE PATENTSCHRIFT

(11) **EP 1 859 005 B1**
(45) Veröffentlichungstag und Bekanntmachung des Hinweises auf die Patenterteilung: **27.04.2016**
(21) Anmeldenummer: 06723216.5
(22) Anmeldetag: 03.03.2006
(51) Int. Cl.: C09K 11/06, H01L 51/30

(54) **NEUE MATERIALIEN FÜR ORGANISCHE ELEKTROLUMINESZENZVORRICHTUNGEN**
NOVEL MATERIALS FOR ORGANIC ELECTROLUMINESCENT DEVICES
NOUVEAUX MATERIAUX POUR DES DISPOSITIFS ELECTROLUMINESCENTS ORGANIQUES

(30) Priorität: 16.03.2005 EP 05005709
(43) Veröffentlichungstag der Anmeldung: 28.11.2007
(73) Patentinhaber: Merck Patent GmbH, 64293 Darmstadt (DE)
(72) Erfinder: STOESSEL, Philipp, 60487 Frankfurt/Main (DE); BREUNING, Esther, 65527 Niedernhausen (DE); PARHAM, Amir, 65929 Frankfurt/Main (DE)
(86) Internationale Anmeldenummer: PCT/EP2006/001991
(87) Internationale Veröffentlichungsnummer: WO 2006/097208

(56) Entgegenhaltungen:
- KOEPF M ET AL: TETRAHEDRON LETTERS, ELSEVIER, AMSTERDAM, NL, Bd. 46, Nr. 1, 3. Januar 2005 (2005-01-03), Seiten 139-142, XP004668577 ISSN: 0040-4039
- CHAMBRON J-C ET AL: COMPTES RENDUS DE L ACADEMIE DES SCIENCES: SERIE II: MECANIQUE- PHYSIQUE-CHIMIE-ASTRONOMIE, EDITIONS SCIENTIFIQUES & MEDICALES ELSEVIER, FR, Bd. 323, Nr. 7, 3. Oktober 1996 (1996-10-03), Seiten 483-492, XP000632253 ISSN: 1251-8069
- ARMAROLI N ET AL: JOURNAL OF THE CHEMICAL SOCIETY. FARADAY TRANSACTIONS, ROYAL SOCIETY OF CHEMISTRY, CAMBRIDGE, GB, Bd. 93, Nr. 23, 7. Dezember 1997 (1997-12-07), Seiten 4145-4150, XP000725090 ISSN: 0956-5000
- ANDERSSON ET AL: J. AM. CHEM. SOC., Bd. 124, Nr. 16, 24. April 2002 (2002-04-24), Seiten 4347-4362, XP002385223 ISSN: 0002-7863
- CHAMBRON ET AL: J. AM. CHEM. SOC., Bd. 115, Nr. 16, 11. August 1993 (1993-08-11), Seiten 7419-7425, XP002385224 ISSN: 0002-7863
- DATABASE WPI Section Ch, Week 200430 Derwent Publications Ltd., London, GB; Class E13, AN 2004-329352 XP002385227 -& WO 2004/018587 A1 (IDEMITSU KOSAN CO LTD) 4. März 2004 (2004-03-04)

## Beschreibung

Die vorliegende Erfindung betrifft neue Materialien, die in organischen elektronischen Vorrichtungen, insbesondere Elektrolumineszenzvorrichtungen, verwendet werden können und die Derivate kondensierter aromatischer Systeme sind.

In einer Reihe verschiedenartiger Anwendungen, die im weitesten Sinne der Elektronikindustrie zugerechnet werden können, ist der Einsatz organischer Halbleiter als funktionelle Materialien seit geraumer Zeit Realität bzw. wird in naher Zukunft erwartet, beispielsweise bei den organischen Elektrolumineszenzvorrichtungen (OLEDs).

Allerdings zeigen diese Vorrichtungen immer noch erhebliche Probleme, die einer dringenden Verbesserung bedürfen:
1. Die operative Lebensdauer ist insbesondere bei blauer Emission immer noch gering, so dass bis dato nur einfache Anwendungen kommerziell realisiert werden konnten.
2. Es werden teilweise Mischungen isomerer Verbindungen verwendet, die unterschiedliche physikalische Eigenschaften (Glasübergangstemperatur, Glasbildungseigenschaften, Absorption, Photolumineszenz) aufweisen können. Da diese Stereoisomeren teilweise auch bei der Verarbeitungstemperatur unterschiedliche Dampfdrücke aufweisen, ist keine einheitliche, reproduzierbare Herstellung der organischen elektronischen Vorrichtung möglich. Dieses Problem wird beispielsweise in der nicht offen gelegten Anmeldung EP 04026402.0 ausführlich beschrieben.
3. Die verwendeten Verbindungen sind teilweise nur schwer in gängigen organischen Lösemitteln löslich, was ihre Reinigung bei der Synthese, aber auch die Reinigung der Anlagen bei der Herstellung der organischen elektronischen Vorrichtungen erschwert.

Als nächstliegender Stand der Technik kann die Verwendung verschiedener kondensierter Aromaten, insbesondere Anthracen- oder Pyrenderivate, als Host-Materialien vor allem für blau emittierende Elektrolumineszenzvorrichtungen genannt werden. Als Host-Material gemäß dem Stand der Technik ist 9,10-Bis(2-naphthyl)anthracen (US 5935721) bekannt. Weitere Anthracen-Derivate, die sich als Host-Materialien eignen, sind beispielsweise in WO 01/076323, in WO 01/021729, in WO 04/013073, in WO 04/018588, in WO 03/087023 oder in WO 04/018587 beschrieben. Host-Materialien, basierend auf aryl-substituierten Pyrenen und Chrysenen, werden in WO 04/016575 beschrieben, wobei hier prinzipiell auch entsprechende Anthracen- und Phenanthren-Derivate mit umfasst sind. In WO 03/095445 und in CN 1362464 werden 9,10-Bis(1-naphthyl)anthracen-Derivate für die Verwendung in OLEDs beschrieben. Auch wenn mit diesen Verbindungen bereits gute Ergebnisse erzielt werden, wäre es wünschenswert, verbesserte Host-Materialien zur Verfügung zu haben. Insbesondere problematisch sind die oben genannten Verbindungen, wenn diese Atropisomere bilden und dadurch bei der Device-Herstellung zu schlecht reproduzierbaren Ergebnissen führen.

Der oben aufgeführte Stand der Technik belegt, dass das Host-Material eine entscheidende Rolle bei der Funktion organischer Elektrolumineszenzvorrichtungen spielt. Daher sollte es möglich sein, durch Optimierung der Materialien die Eigenschaften der organischen elektronischen Vorrichtungen weiter zu verbessern. Es besteht also weiterhin ein Bedarf an Materialien, insbesondere Host-Materialien für blau emittierende OLEDs, die in organischen elektronischen Vorrichtungen zu guten Effizienzen und gleichzeitig zu hohen Lebensdauern führen und die bei der Herstellung und beim Betrieb der Vorrichtungen zu reproduzierbaren Ergebnissen führen. Überraschend wurde gefunden, dass organische elektronische Vorrichtungen, die Makrocyclen mit bestimmten kondensierten Aromaten enthalten, deutliche Verbesserungen gegenüber dem Stand der Technik aufweisen. Mit diesen Materialien ist eine Steigerung der Effizienz und der Lebensdauer in der organischen elektronischen Vorrichtung im Vergleich zu Materialien gemäß dem Stand der Technik möglich. Da diese Materialien keine Atropisomerie zeigen können, ist damit weiterhin die reproduzierbare Herstellung der organischen elektronischen Vorrichtungen möglich. Diese Materialien und deren Verwendung in organischen elektronischen Vorrichtungen sind daher Gegenstand der vorliegenden Erfindung.

In JP 05140145 sind bestimmte Makrocyclen für die Verwendung in OLEDs beschrieben. Diese Verbindungen sind als lumineszierende Materialien oder als Lochtransportverbindungen beschrieben. Die Eignung derartiger Makrocyclen als Host-Materialien geht aus dieser Anmeldung nicht hervor.

WO 2004/018587 A1 offenbart Anthracenderivate mit sperrigen aromatischen Substituenten, die in Elektrolumineszenzvorrichtungen eingesetzt werden.

Gegenstand der Erfindung sind Verbindungen gemäß Formel (1), 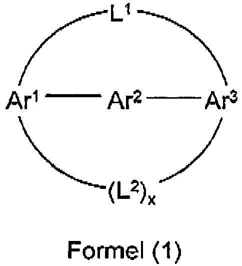 wobei die verwendeten Symbole und Indizes definiert sind wie in Anspruch 1.

Bevorzugt weist die Verbindung gemäß Formel (1) eine Glasübergangstemperatur T_{g} von größer 70 °C auf, besonders bevorzugt größer 100 °C, ganz besonders bevorzugt größer 130 °C.

Ein aromatisches Ringsystem im Sinne dieser Erfindung enthält 6 bis 40 C-Atome im Ringsystem. Ein heteroaromatisches Ringsystem im Sinne dieser Erfindung enthält 2 bis 40 C-Atome und mindestens ein Heteroatom im Ringsystem, mit der Maßgabe, dass die Summe der C-Atome und Heteroatome mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine kurze, nicht-aromatische Einheit (weniger als 10 % der von H verschiedenen Atome, bevorzugt weniger als 5 % der von H verschiedenen Atome), wie z. B. ein sp³-hybridisiertes C-, N- oder O-Atom, unterbrochen sein können. So sollen beispielsweise auch Systeme wie 9,9'-Spirobifluoren, 9,9-Diarylfluoren, Triarylamin, Diarylether, etc. als aromatische Ringsysteme im Sinne dieser Erfindung verstanden werden. Dabei kann ein Teil des aromatischen oder heteroaromatischen Ringsystems auch eine kondensierte Gruppe im Sinne der folgenden Definition sein.

Unter einer kondensierten Aryl- oder Heteroarylgruppe im Sinne dieser Erfindung wird ein Ringsystem mit 9 bis 40 aromatischen Ringatomen verstanden, in dem mindestens zwei aromatische oder heteroaromatische Ringe miteinander "verschmolzen", d. h. durch Anellierung aneinander kondensiert sind, also mindestens eine gemeinsame Kante und ein gemeinsames aromatisches π-Elektronensystem aufweisen. Diese Ringsysteme können durch R substituiert oder unsubstituiert sein. Beispiele für kondensierte aromatische oder heteroaromatische Ringsysteme sind Naphthalin, Chinolin, Anthracen, Phenanthren, Pyren, Perylen, Chrysen, Acridin, etc., während beispielsweise Biphenyl keine kondensierte Arylgruppe darstellt, da dort keine gemeinsame Kante zwischen den beiden Ringsystemen vorliegt. Auch Fluoren stellt beispielsweise kein kondensiertes aromatisches Ringsystem dar, da die beiden Phenyl-Einheiten dort kein gemeinsames aromatisches Ringsystem ausbilden.

Unter benachbarten Positionen im Sinne dieser Erfindung, die für die Verknüpfung von Ar² ausgenommen sind, werden Positionen an zwei direkt benachbarten C-Atomen am Aromaten verstanden. Unter der peri-Position im Sinne dieser Erfindung wird die 1,8-Position an Naphthalin bzw. vergleichbare Positionen an anderen kondensierten Aryl- oder Heteroarylgruppen verstanden.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, besonders bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer C₁- bis C₄₀-Alkoxygruppe werden besonders bevorzugt Methoxy, Ethoxy, n-Propoxy, i-Propoxy, n-Butoxy, i-Butoxy, s-Butoxy, t-Butoxy oder 2-Methylbutoxy verstanden. Unter einem aromatischen oder heteroaromatischen Ringsystem mit 5-30 aromatischen Ringatomen, welches noch jeweils mit den oben genannten Resten R substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Phenanthren, Pyren, Chrysen, Perylen, Fluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Bevorzugt enthält die kondensierte Aryl- oder Heteroarylgruppe Ar² drei, vier, fünf oder sechs aromatische oder heteroaromatische Einheiten, die jeweils über eine oder mehrere gemeinsame Kanten einander ankondensiert sind und dadurch ein gemeinsames aromatisches π-Elektronensystem ausbilden und die durch R substituiert oder unsubstituiert sein können. Besonders bevorzugt enthält die kondensierte Aryl- oder Heteroarylgruppe Ar² drei, vier oder fünf aromatische oder heteroaromatische Einheiten, insbesondere drei oder vier aromatische oder heteroaromatische Einheiten, die jeweils über eine oder mehrere gemeinsame Kanten einander ankondensiert sind und dadurch ein gemeinsames aromatisches System ausbilden und die durch R substituiert oder unsubstituiert sein können. Bevorzugt sind die einander ankondensierten aromatischen und heteroaromatischen Einheiten ausgewählt aus Benzol, Pyridin, Pyrimidin, Pyrazin und Pyridazin, die durch R substituiert oder unsubstituiert sein können, besonders bevorzugt Benzol und Pyridin, ganz besonders bevorzugt ist Benzol. Im folgenden Schema 1 wird schematisch am Beispiel des Anthracens dargestellt, was unter aromatischen Einheiten und gemeinsamen Kanten in einer kondensierten Arylgruppe verstanden wird.

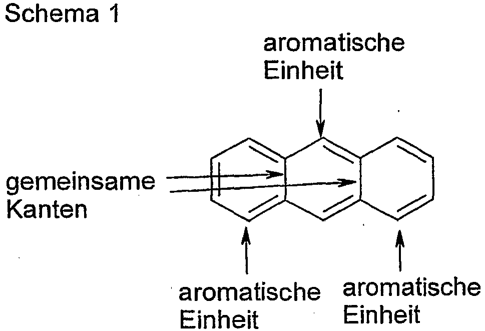

Dabei erfolgt die Verknüpfung der beiden Gruppen Ar¹ und Ar³ nicht über benachbarte Positionen und nicht über peri-Positionen an Ar². Bevorzugt erfolgt die Verknüpfung derart, dass mindestens vier aromatische Ringatome der Einheit Ar² zwischen der Verknüpfung von Ar¹ und Ar³ liegen.

Die kondensierten Aryl- oder Heteroarylgruppen Ar² sind besonders bevorzugt ausgewählt aus der Gruppe bestehend aus Anthracen, Acridin, Phenanthren, Pyren, Naphthacen, Chrysen, Pentacen und Perylen, die gegebenenfalls durch R substituiert sein können. Die Substitution mit R kann sinnvoll sein, um besser lösliche Verbindungen zu erhalten. Besonders bevorzugt sind die kondensierten aromatischen Ringsysteme ausgewählt aus der Gruppe bestehend aus Anthracen, Phenanthren, Pyren oder Perylen, insbesondere Anthracen oder Pyren, die gegebenenfalls durch R substituiert sein können. Dabei erfolgt die Verknüpfung der Einheiten Ar¹ und Ar³ am Anthracen bevorzugt über die 1,5-Position, die 9,10-Position, die 2,6-Position oder über die 1,4-Position, besonders bevorzugt über die 9,10-Position. Die Verknüpfung am Pyren erfolgt bevorzugt über die 1,6-, die 1,8-, die 1,3- oder die 2,7-Position, besonders bevorzugt über die 1,6- oder über die 2,7-Position. Die Verknüpfung am Phenanthren erfolgt bevorzugt über die 2,7-, die 3,6-, die 2,9- oder die 2,10-Position, besonders bevorzugt über die 2,7- oder über die 3,6-Position. Die Verknüpfung am Perylen erfolgt bevorzugt über die 3,9-, die 3,10-, die 3,8- oder die 2,8-Position, besonders bevorzugt über die 3,9- oder über die 3,10-Position. Die Positionen an diesen kondensierten Arylgruppen sind im folgenden Schema 2 abgebildet.

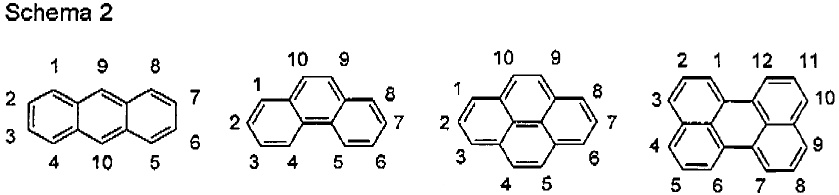

Bevorzugte Gruppen Ar¹ und Ar³ sind, gleich oder verschieden bei jedem Auftreten, aromatische oder heteroaromatische Ringsysteme mit 5 bis 16 aromatischen Ringatomen, ganz besonders bevorzugt mit 6 bis 14 aromatischen Ringatomen. Diese können jeweils durch R substituiert oder unsubstituiert sein. Insbesondere bevorzugt sind aromatische Ringsysteme, die keine aromatischen Heteroatome enthalten. In einer besonders bevorzugten Ausführungsform der Erfindung erfolgt die Verknüpfung der Brücke L¹ und gegebenenfalls der Brücke L² an Ar¹ bzw. Ar³ in ortho-Position zur Verknüpfung mit Ar². Dabei wird unter der ortho-Position, analog zum Benzol, die Position an direkt benachbarten Kohlenstoffatomen verstanden.

Bevorzugt sind die Gruppen Ar¹ und Ar³ gleich gewählt. Diese Bevorzugung ist durch die leichtere synthetische Zugänglichkeit der Verbindungen zu begründen.

Bevorzugt ist für die bivalenten Brücken L¹ und L² die Kombination aus Alkenen und Aromaten bzw. Heteroaromaten. Weiterhin bevorzugt sind Arylamine, Aryloxygruppen oder Alkoxygruppen. Dabei können die Brücken jeweils auch durch die oben genannten Reste R substituiert sein.

Die Brücken L¹ und L² sind besonders bevorzugt ausgewählt aus bivalenten geradkettigen Alkylengruppen, enthaltend 1 bis 10 C-Atome, oder verzweigten oder cyclischen Alkylengruppen, enthaltend 3 bis 10 C-Atome, bivalenten Alkoxy- bzw. Bialkoxygruppen mit 1 bis 10 C-Atomen, bivalenten Alkengruppen, bivalenten aromatischen Ringsystemen mit 6 bis 20 C-Atomen, bivalenten heteroaromatischen Ringsystemen mit 2 bis 20 C-Atomen oder einer Kombination aus zwei, drei oder vier dieser Systeme. Dabei können diese Gruppen auch durch die oben genannten Reste R substituiert sein. Ganz besonders bevorzugt sind die Brücken L¹ und L² ausgewählt aus bivalenten geradkettigen Alkylengruppen, enthaltend 1 bis 8 C-Atome, oder verzweigten oder cyclischen Alkylengruppen, enthaltend 3 bis 10 C-Atome, bivalenten Alkoxy- bzw. Bialkoxygruppen mit 1 bis 8 C-Atomen, bivalenten Alkengruppen, bivalenten aromatischen Ringsystemen mit 6 bis 18 C-Atomen, bivalenten heteroaromatischen Ringsystemen mit 3 bis 18 C-Atomen oder einer Kombination aus zwei oder drei dieser Systeme.

Dabei wird die Länge der Brücke bevorzugt so gewählt, dass weitgehend spannungsfreie Systeme entstehen. Je nach Länge der Brücke können dabei Systeme entstehen, in denen eine freie Rotation der Gruppe Ar² um die Bindung zu Ar¹ und Ar³ möglich ist oder in denen eine freie Rotation der Gruppe Ar² nicht möglich ist.

Dabei wird die Brückenlänge bevorzugt so gewählt, dass die Brücke in der direkten Verbindung 4 bis 20 Brückenatome enthält. Unter der Brückenlänge bzw. der direkten Verbindung wird der kürzeste Pfad entlang den Brückenatomen zwischen den Verknüpfungsstellen verstanden. Dabei ist zu beachten, dass die Anzahl der Brückenatome größer ist als die Brückenlänge, da bei der Brückenlänge nur die Atome der direkten Verbindungen betrachtet werden. Ist Ar² eine 9,10- oder 1,4-verknüpfte Anthracen-Einheit, so enthält die Brücke bevorzugt 4 bis 14 Brückenatome, besonders bevorzugt 5 bis 8 Brückenatome. Ist Ar² eine Pyren-Einheit oder eine 2,7-verknüpfte Phenanthren-Einheit, so enthält die Brücke bevorzugt 5 bis 16 Brückenatome, besonders bevorzugt 7 bis 14 Brückenatome. Ist Ar² eine 3,6-verknüpfte Phenanthren-Einheit, so enthält die Brücke bevorzugt 4 bis 12 Brückenatome, besonders bevorzugt 4 bis 10 Brückenatome.

In einer bevorzugten Ausführungsform der Erfindung ist der Index x = 0, d. h. es ist nur eine Brücke L¹ vorhanden. Dies kann bevorzugt sein, da dadurch Verbindungen mit geringerem Molekulargewicht entstehen, die leichter verdampft werden können bzw. besser löslich sind.

In einer weiteren bevorzugten Ausführungsform der Erfindung ist der Index x =1, d. h. es sind zwei Brücken L¹ und L² in der Struktur vorhanden. Dies kann bevorzugt sein, da sich derartige Verbindungen in manchen Fällen leichter synthetisieren lassen.

Die Verbindungen gemäß Formel (1) bzw. die Brücke(n) können dabei auch geladen sein.

Beispiele für geeignete Verbindungen gemäß Formel (1) sind die im Folgenden abgebildeten Strukturen (1) bis (82).

| | |
|---|---|
| 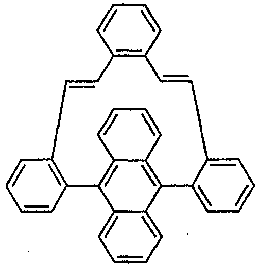 | 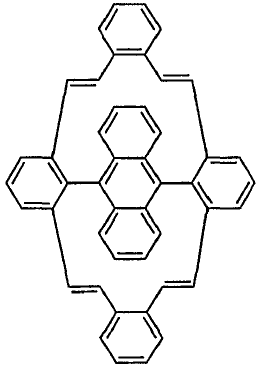 |
| (1) | (2) |
| 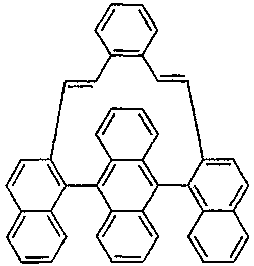 | 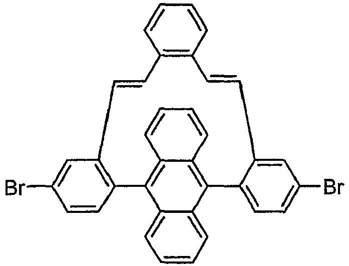 |
| (3) | (4) |
| 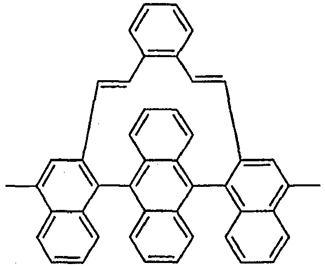 | 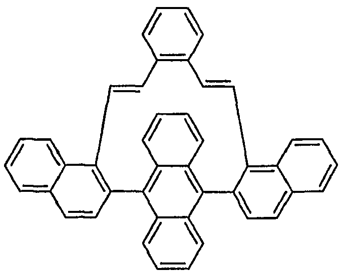 |
| (5) | (6) |
| 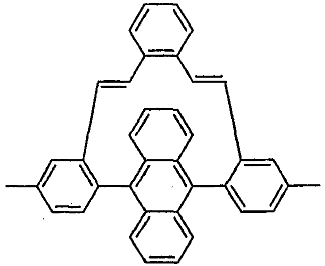 | |
| (7) | |
| 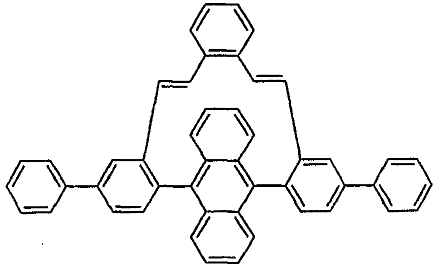 | |
| (8) | |
| 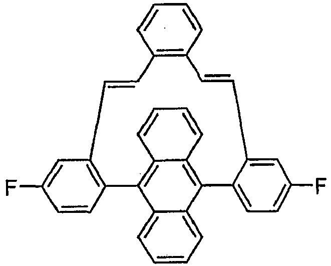 | |
| (9) | |
| 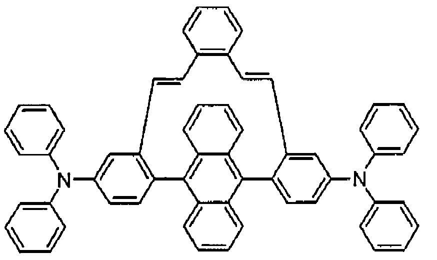 | |
| (10) | |
| 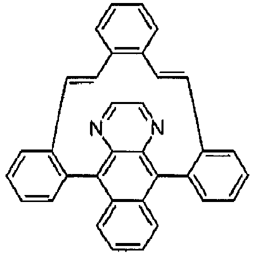 | |
| (11) | |
| 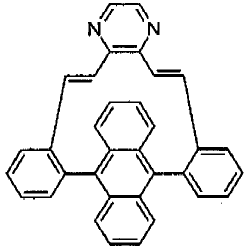 | 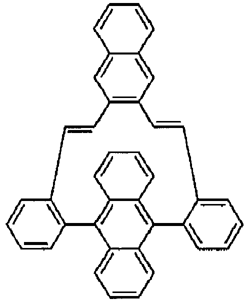 |
| (13) | (14) |
| 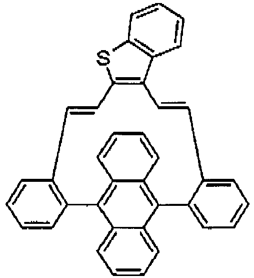 | 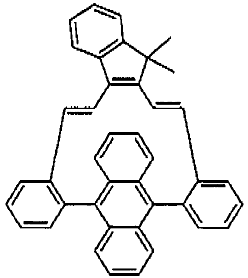 |
| (15) | (16) |
| 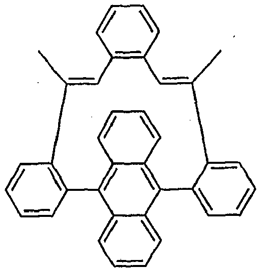 | 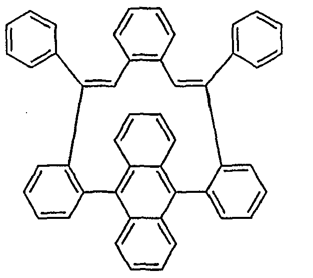 |
| (17) | (18) |
| 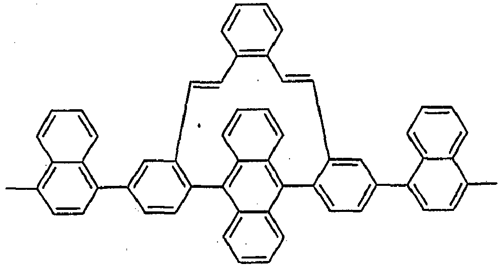 | |
| (19) | |
| 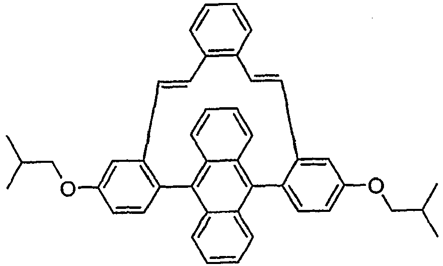 | |
| (20) | |
| 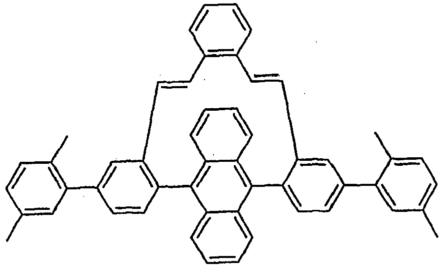 | 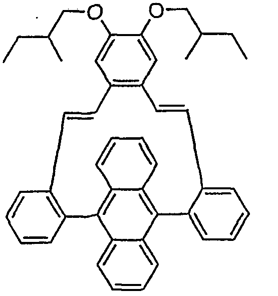 |
| (21) | (22) |
| 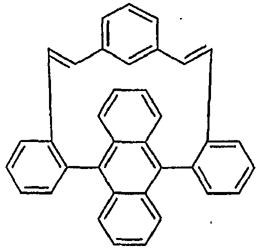 | 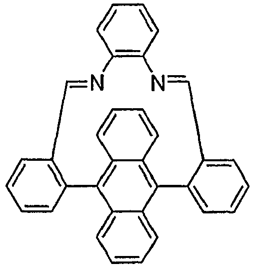 |
| (23) | (24) |
| 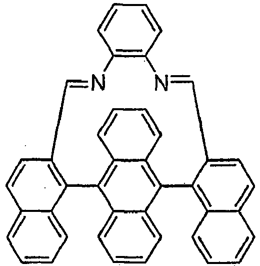 | 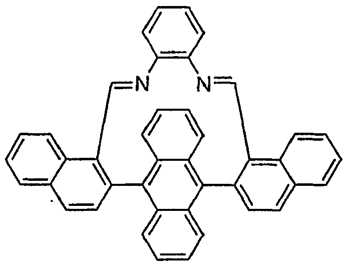 |
| (25) | (26) |
| 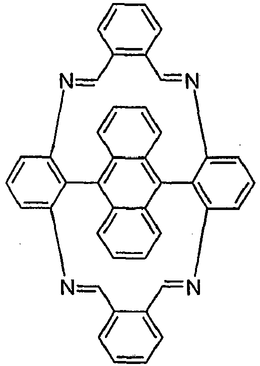 | 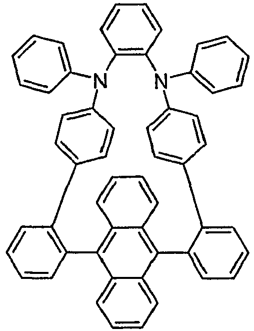 |
| (27) | (28) |
| 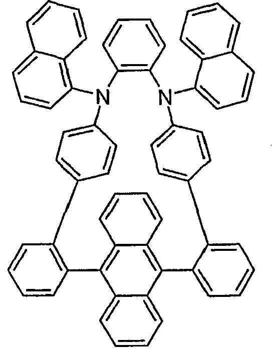 | 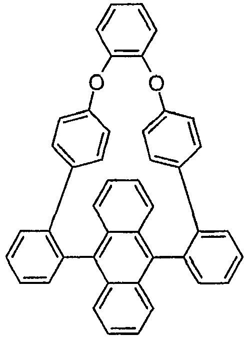 |
| (29) | (30) |
| 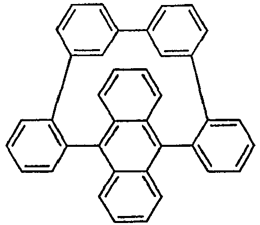 | 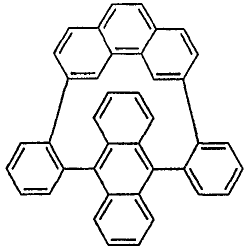 |
| (31) | (32) |
| 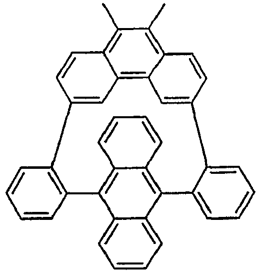 | 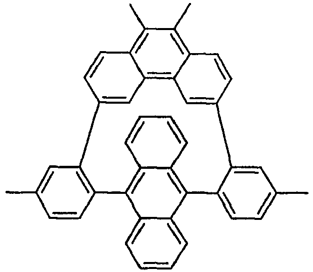 |
| (33) | (34) |
| 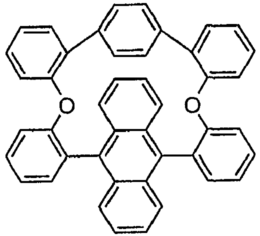 | 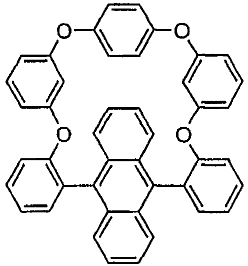 |
| (35) | (36) |
| 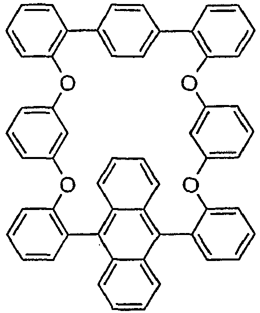 | 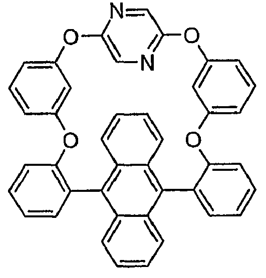 |
| (37) | (38) |
| 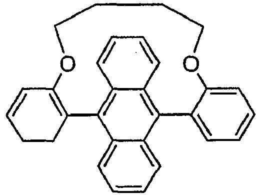 | 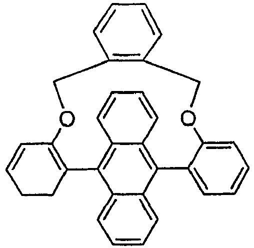 |
| (39) | (40) |
| 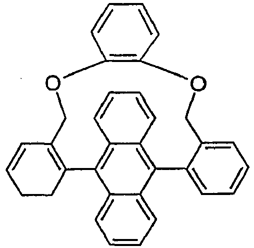 | 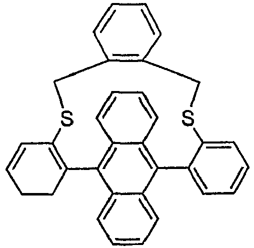 |
| (41) | (42) |
| 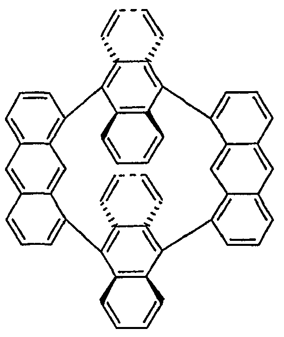 | 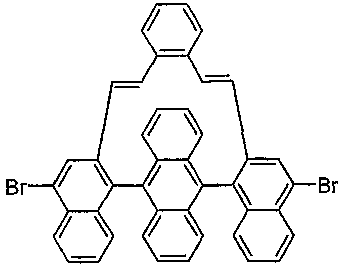 |
| (43) | (44) |
| 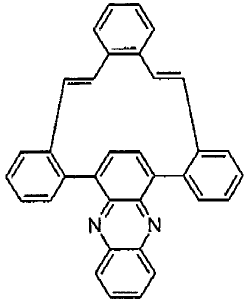 | 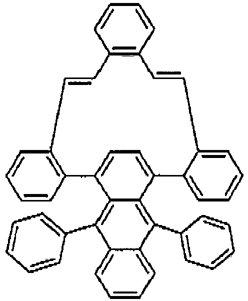 |
| (45) | (46) |

| | |
|---|---|
| 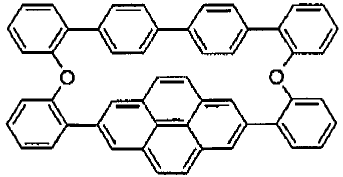 | 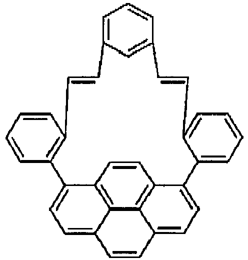 |
| (51) | (52) |
| 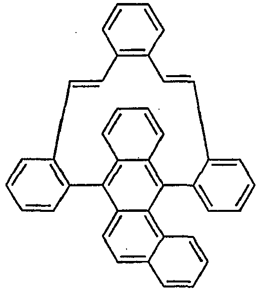 | 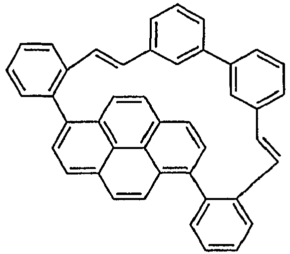 |
| (53) | (54) |
| 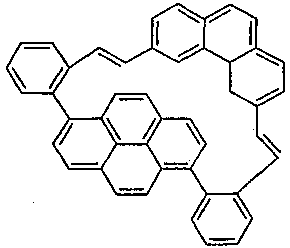 | 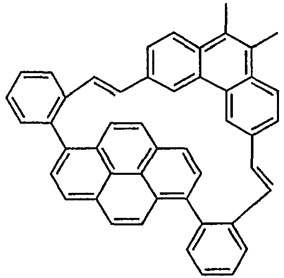 |
| (55) | (56) |
| 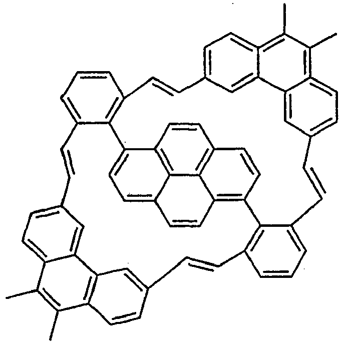 | 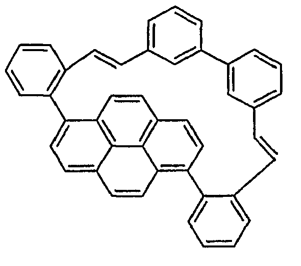 |
| (57) | (58) |
| 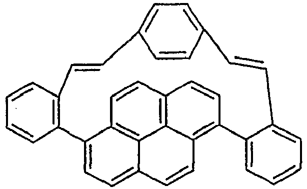 | 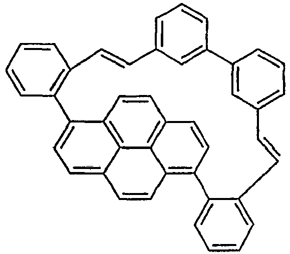 |
| (59) | (60) |
| 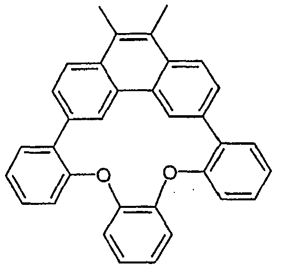 | 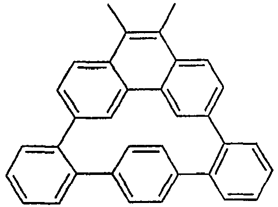 |
| (61) | (62) |
| 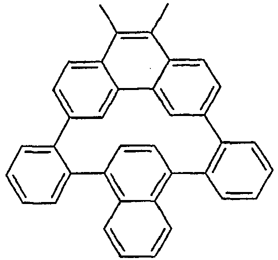 | 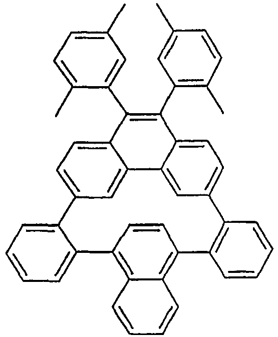 |
| (63) | (64) |
| 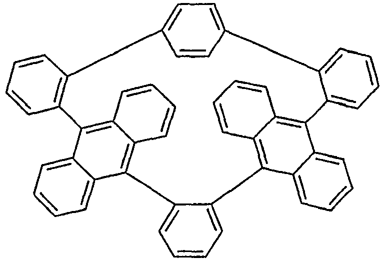 | 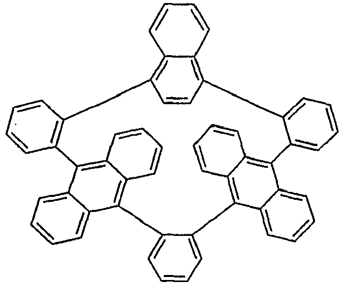 |
| (65) | (66) |
| 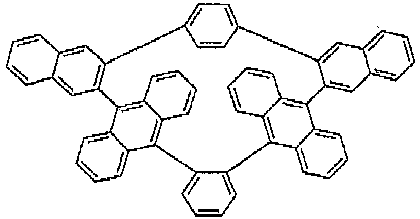 | 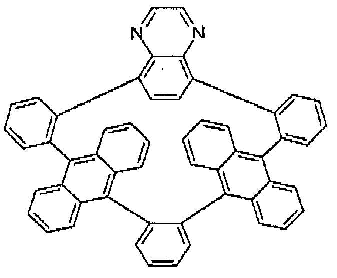 |
| (67) | (68) |
| 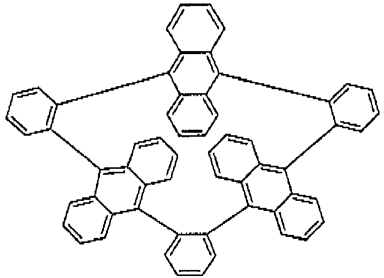 | |
| (69) | |
| | 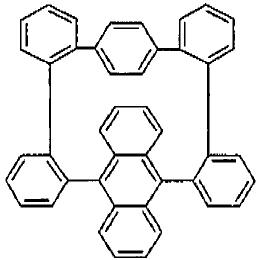 |
| | (72) |
| 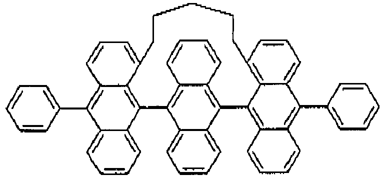 | 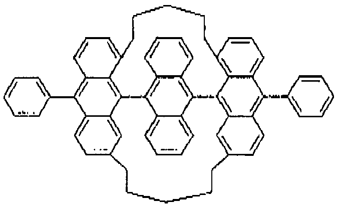 |
| (73) | (74) |
| 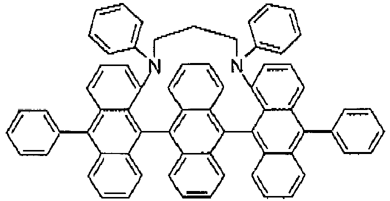 | 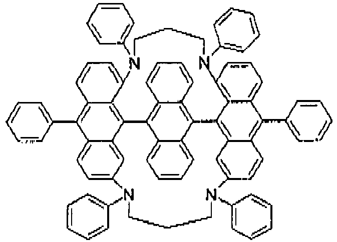 |
| (75) | (76) |
| 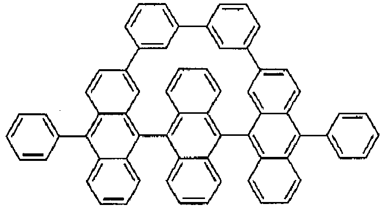 | 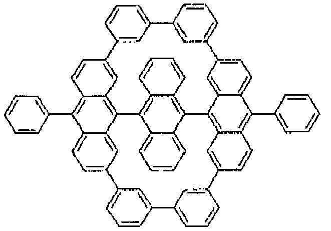 |
| (77) | (78) |
| 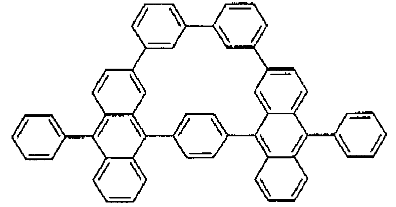 | 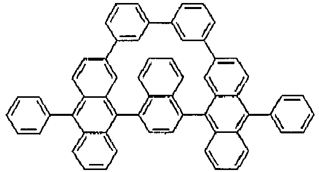 |
| (79) | (80) |

Für die allgemeinen Synthesen von Verbindungen gemäß Formel (1) wird auf B. Dietrich, P. Viout, J.-M. Lehn (Macrocyclic Chemistry, 1992, VCH) verwiesen. Die Synthese von Verbindungen gemäß Formel (1) kann beispielsweise erfolgen, indem zunächst ein System Ar¹-Ar²-Ar³ aufgebaut wird, welches an den aromatischen Gruppen Ar¹ und Ar³ geeignete Funktionalitäten trägt, die eine Bildung der Brücke L¹ bzw. L² ermöglichen.

In einem weiteren Reaktionsschritt kann dann die Brücke L¹ oder auch beide Brücken L¹ und L² eingeführt werden. Falls die Struktur derart ist, dass die Rotation um die Ar¹-Ar²-Bindung und um die Ar²-Ar³-Bindung gehindert ist (Vorliegen von Atropisomeren), kann es sinnvoll sein, zunächst dasjenige Atropisomer zu isolieren, in dem die beiden funktionellen Gruppen auf derselben Seite von Ar² liegen (syn-Isomer). Dies kann beispielsweise durch Umkristallisation oder durch chromatographische Trennung erreicht werden (s. z. B. EP 04026402.0). Dadurch kann die Bildung der Brücke L¹ und der Ringschluss vereinfacht erfolgen. Für die Einführung der Brücke kann es sinnvoll sein, die Reaktion unter Verdünnung durchzuführen, um den Ringschluss zu erleichtern und die Bildung von Oligomeren oder Polymeren zu verhindern. Verschiedene Reaktionstypen, die sich zur Bildung der Brücke L¹ und gegebenenfalls L² eignen, sind beispielsweise die Wittig-Horner-Reaktion, Imin-Bildung, Ether-Bildung, beispielsweise nach Williamson oder Palladium-katalysiert nach Buchwald, Ester-Kondensation nach Claisen, Nitril-Kondensation nach Ziegler, Acyloin-Kondensation, Kondensation von Carbonsäuresalzen des Cers oder des Thoriums nach Ruzicka, Ester-Bildung, Amid-Bildung, 4+2-Cycloaddition, beispielsweise Diels-Alder-Reaktion, Buchwald-Aminierung, Suzuki-Kupplung oder Olefin-Metathese.

Geeignet funktionalisierte Verbindungen gemäß Formel (1), insbesondere bromierte Verbindungen, wie beispielsweise die oben abgebildeten Strukturen (4) und (44), können auch zum Einbau in Polymere verwendet werden.

Gegenstand der Erfindung sind daher weiterhin konjugierte, teilkonjugierte oder nicht-konjugierte Polymere, Oligomere oder Dendrimere, enthaltend Wiederholeinheiten gemäß Formel (1). Diese Wiederholeinheiten können beispielsweise in Polyfluorene (z. B. gemäß EP 842208 oder WO 00/22026), Poly-spirobifluorene (z. B. gemäß EP 707020, EP 894107 oder EP 04028865.6), Poly-para-phenylene (z. B. gemäß WO 92/18552), Poly-dihydrophenanthrene (z. B. gemäß WO 014689), Poly-phenanthrene (z. B. gemäß WO 05/104264), Poly-indenofluorene (z. B. gemäß WO 04/041901 oder WO 04/113412), Poly-carbazole (z. B. gemäß WO 04/070772), Poly-anthracene, Poly-naphthaline oder Polythiophene (z. B. gemäß EP 1028136) einpolymerisiert werden. Auch Polymere mit mehreren dieser Einheiten oder Homopolymere der Wiederholeinheiten gemäß Formel (1) sind möglich.

Gegenstand der Erfindung sind weiterhin Mischungen enthaltend mindestens eine Verbindung gemäß Formel (1) und eine oder mehrere Verbindungen ausgewählt aus der Klasse der Monostyrylamine, der Distyrylamine, der Tristyrylamine, der Tetrastyrylamine und der Arylamine. Unter einem Monostyrylamin wird eine Verbindung verstanden, die eine Styrylgruppe und mindestens ein, bevorzugt aromatisches, Amin enthält. Unter einem Distyrylamin wird eine Verbindung verstanden, die zwei Styrylgruppen und mindestens ein, bevorzugt aromatisches, Amin enthält. Unter einem Tristyrylamin wird eine Verbindung verstanden, die drei Styrylgruppen und mindestens ein, bevorzugt aromatisches, Amin enthält. Unter einem Tetrastyrylamin wird eine Verbindung verstanden, die vier Styrylgruppen und mindestens ein, bevorzugt aromatisches, Amin enthält. Unter einem Arylamin bzw. einem aromatischen Amin im Sinne dieser Erfindung wird eine Verbindung verstanden, die drei aromatische oder heteroaromatische Ringsysteme direkt an den Stickstoff gebunden enthält. Die Styrylgruppen sind besonders bevorzugt Stilbene, die auch noch weiter substituiert sein können. Besonders bevorzugte Dotanden sind ausgewählt aus der Klasse der Tristyrylamine. Beispiele für derartige Dotanden sind substituierte oder unsubstituierte Tristilbenamine oder die Dotanden, die in den nicht offen gelegten Patentanmeldungen DE 102004031000.9, EP 04028407.7 und EP 05001891.0 beschrieben sind.

Gegenstand der Erfindung ist weiterhin die Verwendung der Verbindungen gemäß Formel (1) oder entsprechender Polymere in organischen elektronischen Vorrichtungen.

Nochmals ein weiterer Gegenstand der vorliegenden Erfindung sind organische elektronische Vorrichtungen, enthaltend Anode, Kathode und mindestens eine organische Schicht, welche mindestens eine Verbindung gemäß Formel (1) oder ein entsprechendes Polymer enthält.

Die organischen elektronischen Vorrichtungen sind bevorzugt ausgewählt aus der Gruppe elektronischer Vorrichtungen, bestehend aus organischen und polymeren Leuchtdioden (OLEDs, PLEDs), organischen Feld-Effekt-Transistoren (O-FETs), organischen Dünnfilmtransistoren (O-TFTs), organischen integrierten Schaltungen (O-ICs), organischen Solarzellen (O-SCs), organischen Feld-Quench-Devices (O-FQDs), Photorezeptoren, lichtemittierenden elektrochemischen Zellen (LECs) und organischen Laserdioden (O-Laser). Bevorzugt sind organische und polymere Leuchtdioden.

Die organische elektronische Vorrichtung enthält eine oder mehrere organische Schichten, von denen mindestens eine Schicht mindestens eine Verbindung gemäß Formel (1) enthält. Wenn es sich um eine organische Elektrolumineszenzvorrichtung handelt, ist mindestens eine organische Schicht eine Emissionsschicht. Bei organischen Transistoren ist mindestens eine organische Schicht eine Ladungstransportschicht. In organischen Elektrolumineszenzvorrichtungen können außer der emittierenden Schicht noch weitere Schichten vorhanden sein. Diese können beispielsweise sein: Lochinjektionsschicht, Lochtransportschicht, Ladungsblockierschicht, Elektronentransportschicht und/oder Elektroneninjektionsschicht, die jeweils dotiert oder undotiert sein können. Es sei aber an dieser Stelle darauf hingewiesen, dass nicht notwendigerweise jede dieser Schichten vorhanden sein muss.

Je nach genauer Struktur der Verbindung gemäß Formel (1) kann diese in verschiedenen Funktionen in der organischen elektronischen Vorrichtung verwendet werden, so als Host-Material für Dotanden, die aus dem Singulettzustand oder aus einem Zustand höherer Spinmultiplizität (z. B. dem Triplett-Zustand) Licht emittieren, als Dotand, als Lochtransportmaterial, als Elektronentransportmaterial oder als Lochblockiermaterial. In einer bevorzugten Ausführungsform der Erfindung wird die Verbindung als Host-Material verwendet. Bevorzugte Dotanden sind ausgewählt aus der Gruppe der Monostyrylamine, der Distyrylamine, der Tristyrylamine, der Tetrastyrylamine und der Arylamine, wie vorne beschrieben.

Weiterhin bevorzugt ist eine organische elektronische Vorrichtung, dadurch gekennzeichnet, dass eine oder mehrere Schichten mit einem Sublimationsverfahren beschichtet werden. Dabei werden die Materialien in Vakuum-Sublimationsanlagen bei einem Druck kleiner 10⁻⁵ mbar, bevorzugt kleiner 10⁻⁶ mbar, besonders bevorzugt kleiner 10⁻⁷ mbar aufgedampft.

Bevorzugt ist ebenfalls eine organische elektronische Vorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien in der Regel bei einem Druck zwischen 10⁻⁵ mbar und 1 bar aufgebracht.

Weiterhin bevorzugt ist eine organische elektronische Vorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck oder Offsetdruck, besonders bevorzugt aber LITI (Light Induced Thermal Imaging, Thermotransferdruck) oder InkJet Druck (Tintenstrahl-Druck), hergestellt werden.

Die oben beschriebenen emittierenden Vorrichtungen weisen folgende überraschende Vorteile gegenüber dem Stand der Technik auf:
1. Die Stabilität entsprechender Vorrichtungen ist größer im Vergleich zu Systemen gemäß dem Stand der Technik, was sich vor allem in einer längeren Lebensdauer zeigt.
2. Die Sublimationsstabilität der erfindungsgemäßen Verbindungen ist höher als von Verbindungen gemäß dem Stand der Technik.
3. Im Gegensatz zu bisher verwendeten Verbindungen, die durch ihre schlechte Löslichkeit schwierig zu reinigen waren, sind die Verbindungen gemäß Formel (1) gut löslich und daher einfacher zu reinigen bzw. auch leichter aus Lösung zu verarbeiten.
4. Materialien gemäß dem Stand der Technik bilden teilweise Atropisomere, die zu Problemen mit der Reproduzierbarkeit führen, wie oben bereits erläutert. Durch die Einführung mindestens einer Brücke L¹ wird erfindungsgemäß nur ein Atropisomer verwendet, so dass keine Isomere vorliegen und dadurch die reproduzierbare Herstellung der Vorrichtung möglich ist. Insbesondere ist durch die Einführung dieser Brücke auch keine Reisomerisierung in Lösung bei der Herstellung und Reinigung der Materialien oder im Feststoff oder in der Gasphase bei der Sublimation möglich, so dass hier keine Probleme durch das Vorliegen unterschiedlicher Isomere auftreten können.

### Beispiele:

Die nachfolgenden Synthesen werden, sofern nicht anders angegeben, unter einer Schutzgasatmosphäre durchgeführt. Die Edukte können von den Firmen ALDRICH bzw. ABCR (9,10-Dibromanthracen, 2-Formylbenzolboronsäure, Methoxyphenylboronsäure, 2-Bromphenol, N-Bromsuccinimid (NBS), Tetrakis-tri-phenylphosphino-palladium(0), Anorganika, Lösemittel) bezogen werden. o-Xylylendiphosphonsäure-tetraethylester kann nach DE 19600304 dargestellt werden. 9,10-Bis(2,6-dimethoxyphenyl)anthracen kann nach Zweig et al. (J. Org. Chem. 1967, 32, 1322) dargestellt werden. Anthracen-9-boronsäure und Anthracen-9,10-bisboron-säure können nach Suzuki et al. (Syn. Met. 2004, 143, 89) dargestellt und anschließend mit Ethylenglycol am Wasserabscheider (Schleppmittel Toluol) verestert werden (Ausbeute: Anthracen-9-boronsäureethylenglycol-ester 83.0 %, Anthracen-9,10-bisboronsäure-bisethylenglycolester 33.7 %).

### Beispiel 1: Synthese von Ansaverbindung 1 (A1)

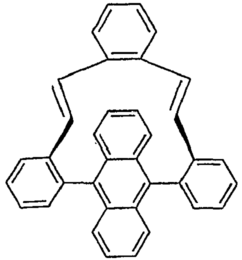

### a) 9,10-Bis(2-formylphenyl)anthracen (Atropisomerengemisch)

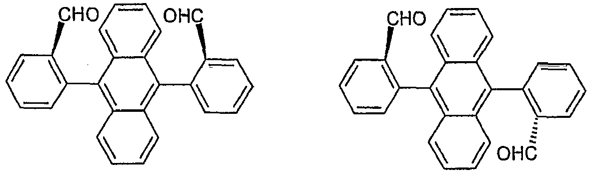

Eine gut gerührte, entgaste Suspension aus 33.6 g (100 mmol) 9,10-Dibromanthracen, 45.0 g (300 mmol) 2-Formylbenzolboronsäure und 55.1 g (520 mmol) Natriumcarbonat in einem Gemisch aus 500 ml Toluol, 150 ml Ethanol und 400 ml Wasser wird mit 2.3 g (2 mmol) Tetrakis-tri-phenylphosphinopalladium(0) versetzt und 60 h unter Rückfluss erhitzt. Nach Erkalten wird die organische Phase abgetrennt, dreimal mit 500 ml Wasser und einmal mit 500 ml gesättigter, wässriger Kochsalzlösung gewaschen und anschließend über Magnesiumsulfat getrocknet. Nach Abfiltrieren des Trockenmittels wird die organische Phase im Vakuum zur Trockene einrotiert. Der so erhaltene ölige Rückstand wird in 300 ml Chloroform gelöst und über eine Kieselgelfritte abgesaugt. Nach Einengen der Chloroformphase im Vakuum wird der breiige Rückstand in 200 ml Ethanol aufgenommen und 1 h bei Raumtemperatur gerührt. Die ausgefallenen Kristalle werden abgesaugt, mit 50 ml Ethanol gewaschen und anschließend im Vakuum getrocknet; Ausbeute: 24.5 g, 63.4 % d. Th., 98 % ig nach ¹H-NMR. Nach ¹H-NMR enthält diese Fraktion zwei Atropisomere, erkennbar an den zwei spektroskopisch aufgelösten Signalen der Formylprotonen, im Verhältnis 1.0 (δ = 9.43 ppm): 1.5 (δ = 9.40 ppm). Die Ethanolmutterlauge wird im Vakuum zu einem Öl aufkonzentriert; Ausbeute: 12.8 g, 33.1 % d. Th., 97 % ig nach ¹H-NMR. Nach ¹H-NMR enthält diese Fraktion zwei Atropisomere im Verhältnis 1.0 (δ = 9.43 ppm) : 2.9 (δ = 9.40 ppm).

### b) Ansaverbindung 1 (A1)

Eine Lösung von 2.08 g (5.5 mmol) o-Xylylendiphosphonsäure-tetraethylester in 500 ml DMF wird bei 0 °C mit 2.11 g (22 mmol) Natrium-tert-butylat versetzt und anschließend 45 min. bei 0 °C gerührt. Die Lösung wird tropfenweise während 1.5 h unter gutem Rühren mit einer Lösung von 1.93 g (5 mmol) 9,10-(2-Formylphenyl)anthracen (Atropisomerengemisch aus a)) in 500 ml DMF versetzt. Die Reaktionsmischung wird weitere 12 h bei Raumtemperatur gerührt und dann tropfenweise mit einer Mischung aus 1000 ml Wasser, 50 ml 1 N HCl und 500 ml Ethanol versetzt. Der ausgefallene Niederschlag wird abfiltriert, dreimal mit je 50 ml Wasser / Ethanol (1:1, v,v), dann dreimal mit je 50 ml Ethanol gewaschen und getrocknet. Der Rückstand wird in 3 ml Essigsäureethylester aufgenommen, mit 15 ml n-Hexan versetzt und 30 min. gerührt. Nach Abfiltrieren von oligo- und polymeren Anteilen wird die Mutterlauge mit n-Hexan / Essigsäureethylester (10:1, v,v) an Kieselgel chromatographiert. Ausbeute: 371 mg, 16.2 % d. Th., Reinheit: 99.0 % nach HPLC. Sublimation: p = 1 x 10⁻⁵ mbar, T = 340 °C.

### Beispiel 2: Synthese von Ansaverbindung 2 (A2)

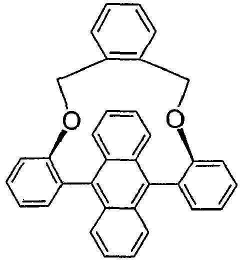

### a) 9,10-Bis(2-hydroxyphenyl)anthracen

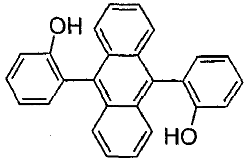

Eine gut gerührte, entgaste Suspension aus 33.6 g (100 mmol) 9,10-Dibromanthracen, 45.6 g (300 mmol) 2-Methoxybenzolboronsäure und 55.1 g (520 mmol) Natriumcarbonat in einem Gemisch aus 500 ml 1,2-Dimethoxyethan, 150 ml Ethanol und 400 ml Wasser wird mit 2.3 g (2 mmol) Tetrakis-tri-phenylphosphinopalladium(0) versetzt und 60 h unter Rückfluss erhitzt. Nach Erkalten wird die organische Phase abgetrennt, dreimal mit 500 ml Wasser und einmal mit 500 ml gesättigter, wässriger Kochsalzlösung gewaschen und anschließend über Magnesiumsulfat getrocknet. Nach Abfiltrieren des Trockenmittels wird die organische Phase im Vakuum zur Trockene eingeengt. Der so erhaltene ölige Rückstand wird in 300 ml Chloroform gelöst und über eine Kieselgelfritte abgesaugt. Nach Einengen der Chloroformphase im Vakuum wird der breiige Rückstand in 500 ml NMP aufgenommen, mit 107.1 g (800 mmol) Lithiumiodid (wasserfrei) versetzt und 24 h bei 190 °C gerührt. Nach Erkalten wird die Reaktionsmischung unter Rühren in 2000 ml 1H HCl eingetragen. Es wird vom ausgefallenen Feststoff abfiltriert und dieser dreimal mit je 200 ml Wasser und dreimal mit je 100 ml Ethanol gewaschen. Abschließend wird je einmal aus DMSO (10 ml / g) und Dioxan (20 ml / g) umkristallisiert und dann im Vakuum getrocknet. Ausbeute: 24.8 g, 68.4 % d. Th., Reinheit: 98.0 % nach HPLC. Die Verbindung zeigt kein Auftreten von Atropisomeren, erkennbar am temperaturunabhängigen ¹H-NMR-Spektrum mit Singulett für die OH-Gruppen, einem AA'BB'-Anteil der Anthracenprotonen und einem ABCD-Anteil der o-Phenylengruppen.

### b) Ansaverbindung 2 (A2)

Ein Gemisch aus 3.62 g (10 mmol) 9,10-Bis(2-hydroxyphenyl)anthracen, 19.74 g (10 mmol) Bariumcarbonat und 500 ml DMSO wird 30 min. auf 100 °C erhitzt. Zu dieser Mischung wird eine Lösung 1.75 g (10 mmol) 1,2-Bischlormethylbenzol in 200 ml DMSO während 2 h zugetropft und abschließend wird 30 min. bei 100 °C nachgerüht. Nach Erkalten wird mit einer Mischung von 1000 ml Wasser und 50 ml 1 N HCl versetzt. Der ausgefallene Niederschlag wird abfiltriert, dreimal mit je 50 ml Wasser / Ethanol (1:1, v,v), dann dreimal mit je 20 ml Ethanol gewaschen und getrocknet. Der Rückstand wird in 5 ml Essigsäureethylester aufgenommen, mit 20 ml n-Hexan versetzt und 30 min. gerührt. Nach Abfiltrieren von oligo- und polymeren Anteilen wird die Mutterlauge mit n-Hexan / Essigsäureethylester (10:1, v,v) an Kieselgel chromatographiert. Ausbeute n. Trocknen im Vakuum: 1.11 g, 23.9 % d. Th., Reinheit: 99.0 % nach HPLC. Sublimation: p = 1 x 10⁻⁵ mbar, T = 340 °C.

### Beispiel 3: Synthese von Ansaverbindung 3 (A3)

### (Vergleichsverbindung außerhalb des Anspruchsumfangs)

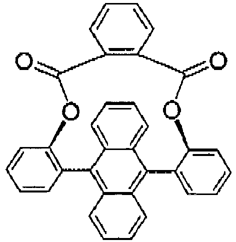

Ein Gemisch aus 3.62 g (10 mmol) 9,10-Bis(2-hydroxyphenyl)anthracen, 3.30 ml (30 mmol) 4-Methylmorpholin, 50 mg 4-Dimethylaminopyridin und 300 ml Dioxan wird auf 70 °C erhitzt. Zu dieser Mischung wird eine Lösung 1.45 ml (10 mmol) Phthaloylchlorid in 200 ml Dioxan während 2 h zugetropft, und abschließend wird noch 30 min. bei 70 °C nachgerührt. Nach Erkalten wird mit einer Mischung aus 1000 ml Wasser und 50 ml 1 N HCl versetzt. Der ausgefallene Niederschlag wird abfiltriert, dreimal mit je 50 ml Wasser / Ethanol (1:1, v,v), dann dreimal mit je 30 ml Ethanol gewaschen und getrocknet. Der Rückstand wird in 5 ml Essigsäureethyl-ester aufgenommen, mit 35 ml n-Hexan versetzt und 30 min. gerührt. Nach Abfiltrieren von oligo- und polymeren Anteilen wird die Mutterlauge mit n-Hexan / Essigsäureethylester (7:1, v,v) an Kieselgel chromato-graphiert. Ausbeute: 1.30 g, 26.4 % d. Th., Reinheit: 99.0 % nach HPLC. Sublimation: p = 1 x 10⁻⁵ mbar, T = 350 °C.

### Beispiel 4: Synthese von Ansaverbindung 4 (A4)

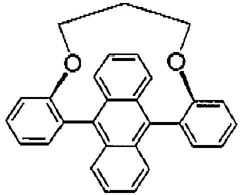

### a) 1,3-Bis(2-bromphenyloxy)propan

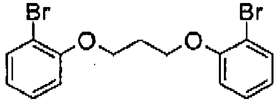

Eine Lösung von 18.2 g (105 mmol) 2-Bromphenol in 200 ml DMF wird unter gutem Rühren portionsweise mit 2.6 g (110) mmol Natriumhydrid versetzt. Nach 15 min. Nachrühren werden 1.5 g (10 mmol) Natriumiodid zugegeben. Dieses Gemisch wird tropfenweise mit einem Gemisch aus 4.8 ml (50 mmol) 1,3-Dibrompropan in 50 ml DMF versetzt und anschließend 60 h bei Raumtemperatur gerührt. Die Raktionsmischung wird tropfenweise mit 5 ml Ethanol versetzt, dann in 1000 ml Wasser eingegossen und dreimal mit 200 ml Dichlormethan extrahiert. Die vereinigten organischen Phasen werden fünfmal mit 500 ml Wasser gewaschen, über MgSO₄ getrocknet und im Vakuum eingeengt. Der verbliebene Schaum wird mit 200 ml n-Heptan ausgerührt, abgesaugt, mit n-Heptan gewaschen und im Vakuum getrocknet. Ausbeute: 15.0 g, 77.7 % d. Th., Reinheit: 98.0 % nach ¹H-NMR.

### b) Ansaverbindung 4 (A4)

Eine Lösung von 3.86 g (10 mmol) 1,3-Bis(2-bromphenyloxy)propan in 1000 ml Diethylether wird unter Rühren tropfenweise mit 8.4 ml n-BuLi (2.5 M in n-Hexan) versetzt. Die Reaktionsmischung wird 2 h bei RT nachgerührt, dann auf -78 °C gekühlt und anschließend tropfenweise mit einer Lösung von 2.10 g (10 mmol) Anthrachinon in 200 ml THF versetzt. Nach langsamem Erwärmen auf Raumtemperatur wird das Lösungsmittel im Vakuum entfernt, der Rückstand wird in 200 ml Eisessig aufgenommen, mit 15.0 g (10 mmol) Natriumiodid und 17.6 g (20mmol) Natriumhypophosphit-Hydrat versetzt und 1 h unter Rückfluss erhitzt. Nach Erkalten wird die Reaktionsmischung in 2 l Wasser eingegossen und dreimal mit je 200 ml Dichlormethan extrahiert. Die vereinigten organischen Phasen werden dreimal mit je 500 ml Wasser gewaschen, anschließend über Magnesiumsulfat getrocknet und im Vakuum zur Trockene eingeengt. Der Rückstand wird in 3 ml Essigsäureethylester aufgenommen, mit 30 ml n-Hexan versetzt und 30 min. gerührt. Nach Abfiltrieren von oligo- und polymeren Anteilen wird die Mutterlauge mit n-Hexan / Essigsäureethylester (10:1, v,v) an Kieselgel chromatographiert. Ausbeute: 601 mg, 14.9 % d. Th., Reinheit: 99.0 % nach HPLC.

### Beispiel 5: Synthese von Ansaverbindung 5 (A5)

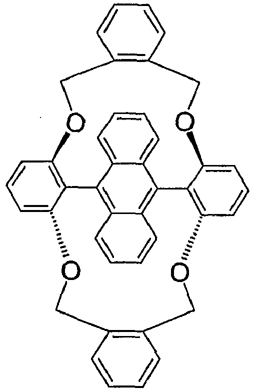

### a) 9,10-Bis(2,6-bishydroxyphenyl)anthracen

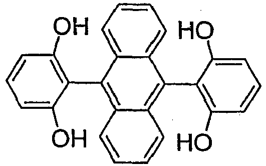

22.5 g (50 mmol) 9,10-Bis(2,6-dimethoxyphenyl)anthracen werden in 500 ml NMP gelöst, mit 107.1 g (800 mmol) Lithiumiodid (wasserfrei) versetzt und 48 h bei 190 °C gerührt. Nach Erkalten wird die Reaktionsmischung unter Rühren in 2000 ml 1N HCl eingetragen. Es wird vom ausgefallenen Feststoff abfiltriert und dieser dreimal mit je 200 ml Wasser und dann dreimal mit je 100 ml Ethanol gewaschen. Abschließend wird aus DMSO (15 ml / g) umkristallisiert und im Vakuum getrocknet. Ausbeute: 16.9 g, 85.7 % d. Th., Reinheit: 99.0 % nach HPLC.

### b) Synthese von Ansaverbindung 5 (A5)

Ein Gemisch von 3.94 g (10 mmol) 9,10-Bis(2,6-bishydroxyphenyl)-anthracen, 39.5 g (20 mmol) Bariumcarbonat und 500 ml DMSO wird 30 min. auf 100 °C erhitzt. Zu dieser Mischung wird eine Lösung 3.85 g (22 mmol) 1,2-Bischlormethylbenzol in 200 ml DMSO während 2 h zugetropft, und abschließend wird 30 min. bei 100 °C nachgerüht. Nach Erkalten wird mit einer Mischung von 1000 ml Wasser und 50 ml 1N HCl versetzt. Der ausgefallene Niederschlag wird abfiltriert, dreimal mit je 50 ml Wasser / Ethanol (1:1, v,v), dann dreimal mit je 50 ml Ethanol gewaschen und getrocknet. Der Rückstand wird mit n-Hexan / Essigsäureethylester (7:1, v,v) an Kieselgel chromatographiert. Ausbeute n. Trocknen im Vakuum: 2.9 g, 48.4 % d. Th., Reinheit: 99.0 % nach HPLC.

### Beispiel 6: Synthese von Ansaverbindung 6 (A6)

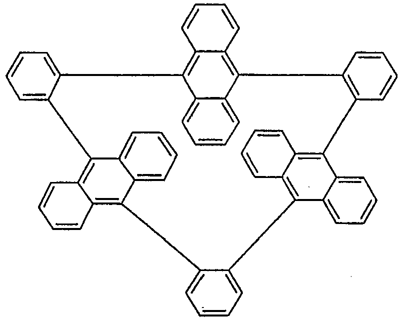

### a) 9,10-Bis(2-bromphenyl)anthracen

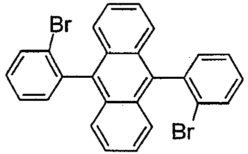

Eine entgaste Suspension von 121 ml (1.0 mol) 1,2-Dibrombenzol, 79.5 g (250 mmol) 9,10-Anthracenbisboronsäurebisethylenglycolester und 157 g Kaliumfluorid (2.7 mol) in einem Gemisch von 1300 ml Dioxan, 350 ml Ethanol und 950 ml Wasser wird mit 2.9 g (2.5 mmol) Tetrakis-triphenyl-phosphinopalladium(0) versetzt und dann 100 h unter Rückfluss erhitzt. Nach Erkalten wird der kristalline Feststoff abgesaugt, dreimal mit je 200 ml Wasser / Ethanol (1:1, v:v), dreimal mit je 100 ml Ethanol gewaschen, im Vakuum getrocknet und aus o-Dichlorbenzol (5 ml / g) umkristallisiert. Ausbeute: 38.8 g, 31.8 % d. Th., Reinheit: 99.0 % nach HPLC.

### b) 1,2-Bis(anthracen-9-yl)benzol

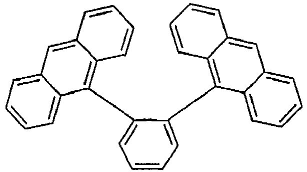

Eine entgaste Suspension von 12.1 ml (100 mmol) 1,2-Dibrombenzol, 74.4 g (300 mmol) 9-Anthracenboronsäureethylenglycolester, 58.1 g Kaliumfluorid (1 mol) in einem Gemisch von 550 ml Dioxan, 150 ml Ethanol, 400 ml Wasser wird mit 1.6 g (1 mmol) Tetrakis-triphenyl-phosphinopalladium(0) versetzt und 100 h unter Rückfluss erhitzt. Nach Erkalten wird der Feststoff abgesaugt, dreimal mit je 200 ml Wasser / Ethanol (1:1, v:v), dreimal mit je 100 ml Ethanol gewaschen und im Vakuum getrocknet. Der Feststoff wird in 1000 ml Essigsäure suspendiert und 1 h unter Rückfluss erhitzt. Man lässt die Suspension auf 90 °C erkalten und saugt schnell über eine Glasfilternutsche (P3) ab. Das Filtrat wird erneut in 700 ml Essigsäure suspendiert, 1 h unter Rückfluss erhitzt und erneut heiß abgesaugt. Der so erhaltene Feststoff wird in 1000 ml Ethanol heiß ausgerührt und im Vakuum getrocknet. Ausbeute: 33.8 g, 78.5 % d. Th., Reinheit: 97 % nach ¹H-NMR.

### c) 1,2-Bis(10-brom-anthracen-9-yl)benzol

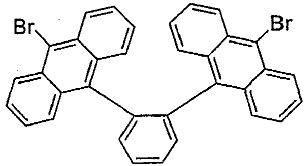

Eine Suspension aus 21.5 (50 mmol) 1,2-Bis(anthracen-9-yl)benzol und 300 g Glasperlen (4 mm Durchmesser) in 500 ml THF wird unter gutem Rühren bei Raumtemperatur unter Lichtausschluss mit 35.6 g (200 mmol) NBS versetzt. Nach 24 h Rühren wird erneut mit 17.8 g (100 mmol) NBS versetzt und weitere 24 h gerührt. Man saugt über eine Schlitzfritte ab, um die Glasperlen zu entfernen und wäscht diese mit 500 ml EtOH nach. Der Feststoff in der Mutterlauge wird abfiltriert, fünfmal mit je 100 ml Ethanol gewaschen und im Vakuum getrocknet. Ausbeute: 22.9 g, 77.8 % d. Th., Reinheit: 97.0 % nach ¹H-NMR (TCE-d2, 90 °C).

### d) Synthese von Ansaverbindung 6 (A6)

Eine auf -78 °C gekühlte Suspension aus 4.88 g (10 mmol) 9,10-Bis(2-bromphenyl)anthracen und 5.88 g 1,2-Bis(10-bromanthracen-9-yl)benzol in 1000 ml THF wird tropfenweise mit 16.8 ml (42 mmol) n-BuLi (2.5 M in n-Hexan) versetzt und 3 h bei -78 °C nachgerührt. Dann gibt man 6.1 g (45 mmol) wasserfreies Kupfer(II)chlorid zu, rührt 1 h bei -78 °C nach, lässt auf Raumtemperatur erwärmen und rührt 16 h bei 50 °C nach. Nach Erkalten und Zugabe von 1000 ml Dichlormethan wird über Kieselgel filtriert, die organische Phase wird fünfmal mit 10 %iger Ammoniaklösung gewaschen, über Magnesiumsulfat getrocknet und im Vakuum eingeengt. Der Rückstand wird in 6 m) Dichlormethan aufgenommen, mit 30 ml n-Hexan versetzt und 30 min. gerührt. Nach Abfiltrieren von oligo- und polymeren Anteilen wird die Mutterlauge mit n-Hexan / Dichlormethan (5:1, v,v) an Kieselgel chromatographiert. Ausbeute: 730 mg, 9.6 % d. Th., Reinheit: 99.0 % nach HPLC. Sublimation: p = 1 x 10⁻⁵ mbar, T = 300 °C.

### Beispiel 7: Herstellung der OLEDs

Die Herstellung von OLEDs erfolgt nach einem allgemeinen Verfahren gemäß WO 04/058911, das im Einzelfall auf die jeweiligen Gegebenheiten (z. B. Schichtdickenvariation, um optimale Effizienz bzw. Farbe zu erreichen) angepasst wird.

In den folgenden Beispielen werden die Ergebnisse verschiedener OLEDs vorgestellt. Der grundlegende Aufbau, die verwendeten Materialien und Schichtdicken, außer der emittierenden Schicht, sind in den allen Beispielen zur besseren Vergleichbarkeit identisch. Analog dem o. g. allgemeinen Verfahren werden OLEDs mit folgendem Aufbau erzeugt:

| | |
|---|---|
| Lochinjektionsschicht (HIL) | 60 nm PEDOT (aus Wasser aufgeschleudert; bezogen von H. C. Starck, Goslar, Deutschland; Poly(3,4-ethylendioxy-2,5-thiophen)) |
| Lochtransportschicht (HTM) | 20 nm NaphDATA (aufgedampft; bezogen von SynTec, Wolfen, Deutschland; 4,4',4"-Tris(N-1-naphthyl-N-phenyl-amino)-triphenylamin) |
| Lochtransportschicht (HTM) | 20 nm S-TAD (aufgedampft; hergestellt nach WO 99/12888; 2,2',7,7'-Tetrakis-(diphenylamino)-spiro-9,9'-bifluoren) |
| Emissionschicht (EML) | siehe Tabelle 1 für Materialien, Konzentration und Schichtdicken |
| Elektronentransportschicht | AlQ₃ 10 nm (aufgedampft: AIQ₃ bezogen von SynTec; Tris(chinolinato)aluminim(III)) |
| Ba-Al (Kathode) | 3 nm Ba, darauf 150 nm Al. |

Diese noch nicht optimierten OLEDs werden standardmäßig charakterisiert; hierfür werden die Elektrolumineszenzspektren, die Effizienz (gemessen in cd/A), die Leistungseffizienz (gemessen in Im/W) in Abhängigkeit der Helligkeit, berechnet aus Strom-Spannungs-Helligkeit-Kennlinien (IUL-Kennlinien), und die Lebensdauer bestimmt. Als Lebensdauer wird die Zeit definiert, nach der die Anfangshelligkeit der OLED bei einer konstanten Stromdichte von 10 mA/cm² auf die Hälfte gesunken ist.

In Tabelle 1 sind die Ergebnisse einiger OLEDs zusammengefasst, wobei jeweils die Zusammensetzung der EML inklusive der Schichtdicken mit aufgeführt ist. Die EMLs enthalten als emittierende Materialien den Dotanden **D1.** Als Hostmaterialien dienen die im Folgenden abgebildeten Verbindungen **V1** und **V2** (Vergleichsmaterialien gemäß dem Stand der Technik) sowie die Ansaverbindungen **A1** bis **A6** (Beispiel 1 bis 6, wobei Verbindung A3 nicht vom Anspruchsumfang umfasst ist).

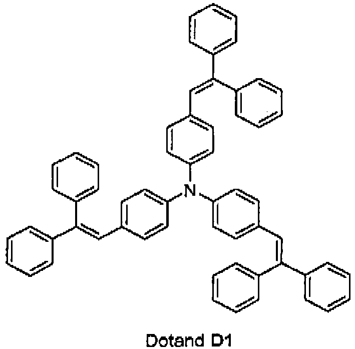

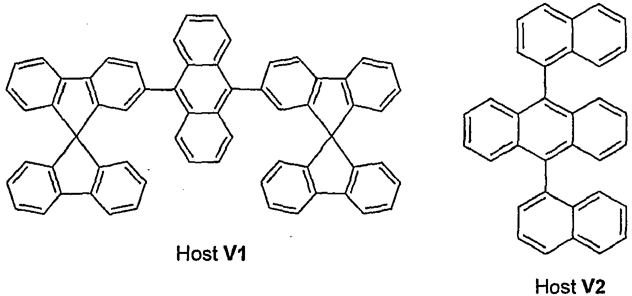

Wie man aus der Tabelle erkennen kann, erhält man mit den erfindungsgemäßen Ansaverbindungen bessere Lebensdauern als mit Hostmaterialien gemäß dem Stand der Technik. Weiterhin ist insbesondere die Ansaverbindung **A6** in Verbindung mit einem blauen Dotanden in der Lage, hocheffiziente weiße Emission aus nur einer Emissionsschicht und bei sehr guter Lebensdauer zu erzeugen.

**Tabelle 1**

| Beispiel | EML | Max Effizienz (cd/A) | Spannung (V) bei 100cd/m² | CIE | Lebensdauer (h) |
|---|---|---|---|---|---|
| Beispiel 7a (Vergleich) | **V1:D1**(5%) (30 nm) | 4.8 | 5.3 | x=0.18; y=0.30 | 3800 |
| Beispiel 7b (Vergleich) | **V2:D1**(5%) (30 nm) | 7.9 | 5.3 | x=0.17;y=0.31 | 15000 |
| Beispiel 7c | **A1:D1**(5%) (30 nm) | 8.3 | 5.1 | x= 0.18; y=0.29 | 17000 |
| Beispiel 7d | **A2:D1**(5%) (30 nm) | 7.7 | 5.2 | x=0.18;y=0.30 | 16500 |
| Beispiel 7e (nicht erfindungsgemäß | **A3: D1** (5 %) (30 nm) | 8.2 | 5.4 | x=0.18; y=0.29 | 15000 |
| Beispiel 7f . | **A4** : **D1** (5 %) (30 nm) | 8.6 | 5.2 | x=0.18; y=0.30 | 19000 |
| Beispiel 7g | **A5 : D1** (5 %) (30 nm) | 8.5 | 4.9 | x=0.17, y=0.29 | 22500 |
| Beispiel 7h | **A6:D1**(5%) (30 nm) | 12.4 | 4.6 | x=0.35; y=0.37 | 21000 |

## Patentansprüche

1. Verbindungen gemäß Formel (1), 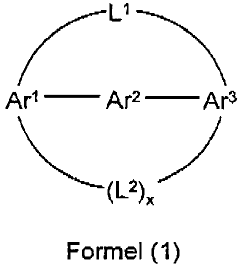 wobei für die verwendeten Symbole und Indizes gilt:
Ar¹, Ar³ ist bei jedem Auftreten gleich oder verschieden ein aromatisches oder heteroaromatisches Ringsystem mit 6 bis 20 aromatischen Ringatomen, welches mit einem oder mehreren Resten R substituiert sein kann;
Ar² ist bei jedem Auftreten gleich oder verschieden eine kondensierte Aryl- oder Heteroarylgruppe mit 14 bis 40 aromatischen Ringatomen, welche mit einem oder mehreren Resten R substituiert sein kann, wobei die kondensierten Aryl- oder Heteroarylgruppen Ar² drei, vier, fünf oder sechs aromatische oder heteroaromatische Einheiten gewählt aus Benzol, Pyrimidin, Pyrazin und Pyridazin enthalten, die jeweils über eine oder mehrere gemeinsame Kanten einander ankondensiert sind und dadurch ein gemeinsames aromatisches System ausbilden, oder Ar² ist Acridin, das mit einem oder mehreren Resten R substituiert sein kann, mit der Maßgabe, dass die Verknüpfung der beiden Gruppen Ar¹ und Ar³ nicht über benachbarte Positionen oder peri-Positionen an Ar² erfolgt;
L¹, L² ist bei jedem Auftreten gleich oder verschieden eine bivalente organische Brücke enthaltend 1 bis 60 C Atome, welche ausgewählt ist aus den Gruppen der Alkene, Aromaten enthaltend 6 bis 40 C-Atome, Heteroaromaten enthaltend 2 bis 40 C-Atome, Imine, Alkoxygruppen, Thioalkoxygruppen, Aryloxygruppen, Thioarylgruppen, Amine, Arylamine, Alkylene und Arylborane oder Kombinationen aus einem oder mehreren dieser Systeme, die mit einem oder mehreren Resten R substituiert sein kann;
R ist bei jedem Auftreten gleich oder verschieden H, F, Cl, Br, I, CN, NO₂ COOR¹, B(OR¹)₂, B(R¹)₂, Si(R¹)₃, eine geradkettige Alkyl- oder Alkoxykette mit 1 bis 40 C-Atomen oder eine verzweigte oder cyclische Alkyl- oder Alkoxykette mit 3 bis 40 C-Atomen, die jeweils durch R¹ substituiert sein kann, in der auch ein oder mehrere nicht benachbarte C-Atome durch N-R¹, O, S, O-CO-O, CO-O, -CR¹=CR¹- oder -C≡C- ersetzt sein können und in der auch ein oder mehrere H-Atome durch F, Cl, Br, I oder CN ersetzt sein können, oder ein aromatisches oder heteroaromatisches Ringsystem mit 5 bis 40 aromatischen Ringatomen, welches durch einen oder mehrere Reste R¹ substituiert sein kann, oder eine Kombination aus zwei, drei oder vier dieser Systeme; dabei können zwei oder mehrere Reste R miteinander auch ein weiteres mono- oder polycyclisches, aliphatisches oder aromatisches Ringsystem bilden;
R¹ ist bei jedem Auftreten gleich oder verschieden H oder ein aliphatischer oder aromatischer Kohlenwasserstoffrest mit 1 bis 20 C-Atomen; dabei können zwei oder mehrere Reste R¹ auch miteinander ein Ringsystem bilden;
x ist bei jedem Auftreten gleich oder verschieden 0 oder 1, wobei x = 0 bedeutet, dass die Brücke L² nicht anwesend ist.

2. Verbindungen gemäß Anspruch 1, **dadurch gekennzeichnet, dass** die kondensierten Aryl- oder Heteroarylgruppen Ar² ausgewählt sind aus der Gruppe bestehend aus Anthracen, Acridin, Phenanthren, Pyren, Naphthacen, Chrysen, Pentacen und Perylen, die durch R substituiert oder unsubstituiert sein können.

3. Verbindungen gemäß Anspruch 1 oder 2, **dadurch gekennzeichnet, dass** die Verknüpfung der beiden Gruppen Ar¹ und Ar³ derart erfolgt, dass mindestens vier aromatische Ringatome der Einheit Ar² zwischen Ar¹ und Ar³ liegen.

4. Verbindungen gemäß einem oder mehreren der Ansprüche 1 bis 3, **dadurch gekennzeichnet, dass** die Verknüpfung der Brücke L¹ und gegebenenfalls der Brücke L² an Ar¹ bzw. Ar³ in ortho-Position zur Verknüpfung mit Ar² erfolgt.

5. Verbindungen gemäß einem oder mehreren der Ansprüche 1 bis 4, **dadurch gekennzeichnet, dass** die Gruppen Ar¹ und Ar³ gleich gewählt sind.

6. Verbindungen gemäß einem oder mehreren der Ansprüche 1 bis 5, **dadurch gekennzeichnet, dass** die Länge der Brücke L¹ und L² so gewählt wird, dass weitgehend spannungsfreie Systeme entstehen.

7. Konjugierte, teilkonjugierte oder nicht-konjugierte Polymere, Oligomere oder Dendrimere, enthaltend Wiederholei nheiten gemäß einem oder mehreren der Ansprüche 1 bis 6.

8. Mischungen enthaltend mindestens eine Verbindung gemäß einem oder mehreren der Ansprüche 1 bis 7 und eine oder mehrere Verbindungen ausgewählt aus der Klasse der Monostyrylamine, der Distyrylamine, der Tristyrylamine, der Tetrastyrylamine und der Arylamine.

9. Verwendung von Verbindungen und Mischungen gemäß einem oder mehreren der Ansprüche 1 bis 8 in organischen elektronischen Vorrichtungen.

10. Organische elektronische Vorrichtungen, enthaltend Anode, Kathode und mindestens eine organische Schicht, welche mindestens eine Verbindung oder eine Mischung gemäß einem oder mehreren der Ansprüche 1 bis 8 enthält.

11. Organische elektronische Vorrichtung gemäß Anspruch 10, ausgewählt aus der Gruppe elektronischer Vorrichtungen, bestehend aus organischen und polymeren Leuchtdioden (OLEDs, PLEDs), organischen Feld-Effekt-Transistoren (O-FETs), organischen Dünnfilmtransistoren (O-TFTs), organischen integrierten Schaltungen (O-ICs), organischen Solarzellen (O-SCs), organischen Feld-Quench-Devices (O-FQDs) und organischen Laserdioden (O-Laser).

12. Organische elektronische Vorrichtung gemäß Anspruch 10 und/oder 11, **dadurch gekennzeichnet, dass** die Verbindung gemäß einem oder mehreren der Ansprüche 1 bis 7 als Host-Material in einer organischen Elektrolumineszenzvorrichtung verwendet wird.

13. Organische elektronische Vorrichtung gemäß Anspruch 12, **dadurch gekennzeichnet, dass** die Dotanden ausgewählt sind aus der Gruppe der Monostyrylamine, der Distyrylamine, der Tristyrylamine, der Tetrastyrylamine und der Arylamine.

## Claims

1. Compounds of the formula (1), 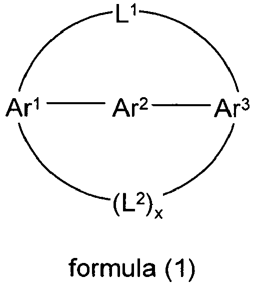 where the following applies to the symbols and indices used:
Ar¹, Ar³ are on each occurrence, identically or differently, an aromatic or heteroaromatic ring system having 6 to 20 aromatic ring atoms, which may be substituted by one or more radicals R;
Ar² is on each occurrence, identically or differently, a condensed aryl or heteroaryl group having 14 to 40 aromatic ring atoms, which may be substituted by one or more radicals R, where the condensed aryl or heteroaryl groups Ar² contain three, four, five or six aromatic or heteroaromatic units selected from benzene, pyrimidine, pyrazine and pyridazine, which are in each case condensed onto one another via one or more common edges and thus form a common aromatic system, or Ar² is acridine, which may be substituted by one or more radicals R, with the proviso that the two groups Ar¹ and Ar³ are not linked via adjacent positions or peri positions on Ar²;
L¹, L² are on each occurrence, identically or differently, a divalent organic bridge containing 1 to 60 C atoms which is selected from the groups of the alkenes, aromatic groups containing 6 to 40 C atoms, heteroaromatic groups containing 2 to 40 C atoms, imines, alkoxy groups, thioalkoxy groups, aryloxy groups, thioaryl groups, amines, arylamines, alkylenes and arylboranes, or combinations of one or more of these systems, which may be substituted by one or more radicals R;
R is on each occurrence, identically or differently, H, F, Cl, Br, I, CN, NO₂, COOR¹, B(OR¹)₂, B(R¹)₂, Si(R¹)₃, a straight-chain alkyl or alkoxy chain having 1 to 40 C atoms or a branched or cyclic alkyl or alkoxy chain having 3 to 40 C atoms, each of which may be substituted by R¹ and in which, in addition, one or more non-adjacent C atoms may be replaced by N-R¹, O, S, O-CO-O, CO-O, -CR¹=CR¹- or -C≡C- and in which, in addition, one or more H atoms may be replaced by F, Cl, Br, I or CN, or an aromatic or heteroaromatic ring system having 5 to 40 aromatic ring atoms, which may be substituted by one or more radicals R¹, or a combination of two, three or four of these systems; two or more radicals R here may also form a further mono- or polycyclic, aliphatic or aromatic ring system with one another;
R¹ is on each occurrence, identically or differently, H or an aliphatic or aromatic hydrocarbon radical having 1 to 20 C atoms; two or more radicals R¹ here may also form a ring system with one another;
x is on each occurrence, identically or differently, 0 or 1, where x = 0 means that bridge L² is not present.

2. Compounds according to Claim 1, **characterised in that** the condensed aryl or heteroaryl groups Ar² are selected from the group consisting of anthracene, acridine, phenanthrene, pyrene, naphthacene, chrysene, pentacene and perylene, each of which may be substituted by R or unsubstituted.

3. Compounds according to Claim 1 or 2, **characterised in that** the two groups Ar¹ and Ar³ are linked in such a way that at least four aromatic ring atoms of the unit Ar² are located between Ar¹ and Ar³.

4. Compounds according to one or more of Claims 1 to 3, **characterised in that** the bridge L¹ and, if present, the bridge L² are linked to Ar¹ and Ar³ in the ortho position to the link to Ar².

5. Compounds according to one or more of Claims 1 to 4, **characterised in that** the groups Ar¹ and Ar³ are selected identically.

6. Compounds according to one or more of Claims 1 to 5, **characterised in that** the length of the bridges L¹ and L² is selected so that substantially strain-free systems are formed.

7. Conjugated, partially conjugated or non-conjugated polymers, oligomers or dendrimers containing recurring units according to one or more of Claims 1 to 6.

8. Mixtures comprising at least one compound according to one or more of Claims 1 to 7 and one or more compounds selected from the class of the monostyrylamines, the distyrylamines, the tristyrylamines, the tetrastyrylamines and the arylamines.

9. Use of compounds and mixtures according to one or more of Claims 1 to 8 in organic electronic devices.

10. Organic electronic device comprising anode, cathode and at least one organic layer which comprises at least one compound or mixture according to one or more of Claims 1 to 8.

11. Organic electronic device according to Claim 10, selected from the group of electronic devices consisting of organic and polymeric light-emitting diodes (OLEDs, PLEDs), organic field-effect transistors (O-FETs), organic thin-film transistors (O-TFTs), organic integrated circuits (O-ICs), organic solar cells (O-SCs), organic field-quench devices (O-FQDs) and organic laser diodes (O-lasers).

12. Organic electronic device according to Claim 10 and/or 11, **characterised in that** the compound according to one or more of Claims 1 to 7 is used as host material in an organic electroluminescent device.

13. Organic electronic device according to Claim 12, **characterised in that** the dopants are selected from the group of the monostyrylamines, the distyrylamines, the tristyrylamines, the tetrastyrylamines and the arylamines.

## Revendications

1. Composés de la formule (1) : 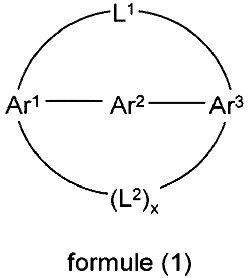 dans laquelle ce qui suit s'applique aux symboles et indices utilisés :
Ar¹, Ar³ sont pour chaque occurrence, de manière identique ou différente, un système de cycle aromatique ou hétéroaromatique comportant 6 à 20 atomes de cycle aromatique, lequel peut être substitué par un radical ou plusieurs radicaux R ;
Ar² est pour chaque occurrence, de manière identique ou différente, un groupe aryle ou hétéroaryle condensé comportant 14 à 40 atomes de cycle aromatique, lequel peut être substitué par un radical ou plusieurs radicaux R, où les groupes aryle ou hétéroaryle condensés Ar² contiennent trois, quatre, cinq ou six unités aromatiques ou hétéroaromatiques choisies parmi benzène, pyrimidine, pyrazine et pyridazine, lesquelles sont dans chaque cas condensées les unes sur les autres via un ou plusieurs bord(s) commun(s) et forment en conséquence un système aromatique commun, ou Ar² est acridine, lequel peut être substitué par un radical ou plusieurs radicaux R, étant entendu que les deux groupes Ar¹ et Ar³ ne sont pas liés via des positions ou des péri-positions adjacentes sur Ar² ;
L¹, L² sont pour chaque occurrence, de manière identique ou différente, un pont organique divalent contenant 1 à 60 atome(s) de C, lequel est choisi parmi les groupes des alcènes, les groupes aromatiques contenant 6 à 40 atomes de C, les groupes hétéroaromatiques contenant 2 à 40 atomes de C, les imines, les groupes alcoxy, les groupes thioalcoxy, les groupes aryloxy, les groupes thioaryle, les amines, les arylamines, les alkylènes et les arylboranes, ou des combinaisons d'un ou de plusieurs de ces systèmes, lesquels peuvent être substitués par un radical ou plusieurs radicaux R ;
R est pour chaque occurrence, de manière identique ou différente, H, F, Cl, Br, I, CN, NO₂ COOR¹, B(OR¹)₂, B(R¹)₂, Si(R¹)₃, une chaîne alkyle ou alcoxy en chaîne droite comportant 1 à 40 atome(s) de C ou une chaîne alkyle ou alcoxy ramifiée ou cyclique comportant 3 à 40 atomes de C, dont chacune peut être substituée par R¹ et où, en outre, un ou plusieurs atome(s) de C non adjacents peut/ peuvent être remplacé(s) par N-R¹, O, S, O-CO-O, CO-O, -CR¹=CR¹- ou -C≡C- et où, en outre, un ou plusieurs atome(s) de H peut/peuvent être remplacé(s) par F, Cl, Br, I ou CN, ou un système de cycle aromatique ou hétéroaromatique comportant 5 à 40 atomes de cycle aromatique, lequel peut être substitué par un radical ou plusieurs radicaux R¹, ou une combinaison de deux, trois ou quatre de ces systèmes ; deux radicaux R ou plus peuvent ici également former un autre système de cycle aliphatique ou aromatique monocyclique ou polycyclique l'un avec l'autre ou les uns avec les autres ;
R¹ est pour chaque occurrence, de manière identique ou différente, H ou un radical hydrocarbone aliphatique ou aromatique comportant 1 à 20 atome(s) de C ; deux radicaux R¹ ou plus peuvent ici également former un système de cycle l'un avec l'autre ou les uns avec les autres ;
x est pour chaque occurrence, de manière identique ou différente, 0 ou 1, où x = 0 signifie qu'un pont L² n'est pas présent.

2. Composés selon la revendication 1, **caractérisés en ce que** les groupes aryle ou hétéroaryle condensés Ar² sont choisis parmi le groupe constitué par anthracène, acridine, phénanthrène, pyrène, naphtacène, chrysène, pentacène et perylène, dont chacun peut être substitué par R ou non substitué.

3. Composés selon la revendication 1 ou 2, **caractérisés en ce que** les deux groupes Ar¹ et Ar³ sont liés de telle sorte qu'au moins quatre atomes de cycle aromatique de l'unité Ar² soient localisés entre Ar¹ et Ar³.

4. Composés selon une ou plusieurs des revendications 1 à 3, **caractérisés en ce que** le pont L¹ et, s'il est présent, le pont L² sont liés à Ar¹ et Ar³, à la position ortho, à la liaison sur Ar².

5. Composés selon une ou plusieurs des revendications 1 à 4, **caractérisés en ce que** les groupes Ar¹ et Ar³ sont choisis de manière identique.

6. Composés selon une ou plusieurs des revendications 1 à 5, **caractérisés en ce que** la longueur des ponts L¹ et L² est choisie de telle sorte que des systèmes sensiblement exempts de contraintes soient formés.

7. Polymères, oligomères ou dendrimères conjugués, partiellement conjugués ou non conjugués contenant des unités récurrentes selon une ou plusieurs des revendications 1 à 6.

8. Mélanges comprenant au moins un composé selon une ou plusieurs des revendications 1 à 7 et un ou plusieurs composé(s) choisi(s) parmi la classe des monostyrylamines, des distyrylamines, des tristyrylamines, des tétrastyrylamines et des arylamines.

9. Utilisation de composés et mélanges selon une ou plusieurs des revendications 1 à 8 dans des dispositifs électroniques organiques.

10. Dispositif électronique organique comprenant une anode, une cathode et au moins une couche organique qui comprend au moins un composé ou mélange selon une ou plusieurs des revendications 1 à 8.

11. Dispositif électronique organique selon la revendication 10, choisi parmi le groupe de dispositifs électroniques constitué par des diodes émettrices de lumière organiques et polymériques (OLED, PLED), des transistors à effet de champ organiques (O-FET), des transistors à film mince organiques (O-TFT), des circuits intégrés organiques (O-IC), des cellules solaires organiques (O-SC), des dispositifs à extinction de champ organiques (O-FQD) et des diodes laser organiques (O-laser).

12. Dispositif électronique organique selon la revendication 10 et/ou 11, **caractérisé en ce que** le composé selon une ou plusieurs des revendications 1 à 7 est utilisé en tant que matériau hôte dans un dispositif électroluminescent organique.

13. Dispositif électronique organique selon la revendication 12, **caractérisé en ce que** les dopants sont choisis parmi le groupe des monostyrylamines, des distyrylamines, des tristyrylamines, des tétrastyrylamines et des arylamines.
